Europäisches Patentamt

European Patent Office

Office européen des brevets

(19)

(11) Publication number: **0 016 578**
**B1**

(12) **EUROPEAN PATENT SPECIFICATION**

(45) Date of publication of patent specification: **02.01.85**

(51) Int. Cl.⁴: **G 01 N 31/22**

(21) Application number: **80300664.2**

(22) Date of filing: **05.03.80**

(54) **Method and kit for the determination of biocide concentration in aqueous systems.**

| | |
|---|---|
| (30) Priority: **10.03.79 GB 7908511** | (73) Proprietor: **The British Petroleum Company p.l.c.**<br>**Britannic House Moor Lane**<br>**London EC2Y 9BU (GB)** |
| (43) Date of publication of application:<br>**01.10.80 Bulletin 80/20** | |
| (45) Publication of the grant of the patent:<br>**02.01.85 Bulletin 85/01** | (72) Inventor: **Franks, Malcolm Carpenter**<br>**The British Petroleum Company Ltd. Chertsey Road**<br>**Sunbury-On-Thames Middlesex TW16 7LN (GB)**<br>Inventor: **Peacock, Daniel John Alan**<br>**The British Petroleum Company Ltd. Chertsey Road**<br>**Sunbury-On-Thames Middlesex TW16 7LN (GB)** |
| (84) Designated Contracting States:<br>**AT BE CH DE FR GB NL SE** | |
| (56) References cited:<br>**GB-A-1 500 850**<br><br>**TEXTIL VEREDLUNG, vol. 11, no. 8, August 1976, pages 345-348, S.V.F.: S.V.C.C., Basel, CH, W. BITTERLI et al.: "Nachweis geringster Mengen Formaldehyd in wässrigen Lösungen - Vergleich zweier spektralkolorimetrischer Methoden zur quantitativen Bestimmung von Formaldehyd in Textilien"**<br>**ANALYTICAL CHEMISTRY, vol. 33, no. 1, January 1961, pages 93-96, U.S.A., E. SAWICKI et al.: "The 3-Methyl-2 benzothiazolone Hydrazone Test"** | (74) Representative: **Eastman, Hugh Leonard et al**<br>**c/o The British Petroleum Company plc Patents Division Chertsey Road**<br>**Sunbury-on-Thames Middlesex TW16 7LN (GB)** |

# 0016 578

**Description**

The present invention relates to a colorimetric method for the determination of aldehyde release biocides when present in aqueous systems, and to a kit for carrying out the method.

Aldehyde release biocides are known and this type of biocide is commonly used to suppress the growth of micro-organisms in aqueous systems. It is not known whether the biocidal activity results from the liberation of an aldehyde by slow chemical release or whether the biocide molecules are able to donate aldehyde by some other reaction, to produce the biocidal activity. For this reason, the aldehyde release biocides are considered to include biocides which are aldehyde donors. For convenience only the term aldehyde release biocide is used in this specification. The aldehyde released by such biocides is usually formaldehyde. Some examples of biocides of this type are those sold under the Trade Marks Grotan, Grotan BK, Bodoxin, Cinon, Vancide TH, Tris Nitro and Giv-Gard DXN. Typically these biocides are formed by a condensation reaction between formaldehyde and an amine, for example monoethanolamine or ethylamine.

Aqueous systems in which biocides of this type are used are, for example, soluble oil/water emulsions or synthetic water based fluids. The aqueous systems are used in metal cutting, grinding, rolling and forming processes or as water based hydraulic fluids in mining, die casting and steel pressing operations. The presence of the biocide maintains the performance and prolongs the effective life of the system.

To be effective in the control of micro-organisms the concentration of the biocide present in the system must be maintained at least at a minimum which is effective in inhibiting growth of contaminant micro-organisms. On the other hand, since these biocides function by the release into the system of an aldehyde, usually formaldehyde, the maximum concentration should be carefully controlled in order to minimise any health hazard to the operating personnel due to the build up of the aldehyde. Aldehydes, and in particular formaldehyde, are recognised allergenic agents. For example, concentrations of formaldehyde in excess of 1000 parts per million are thought to present a potential health hazard to non-sensitive and healthy individuals, (Epstein Arch. Derm. 1966.94.186). It is common practice on the shop floor to add biocide to the aqueous system without regard to the concentration which is actually present. Attempts have been made to devise a method for the rapid determination of biocides in aqueous systems of this type which is suitable for use by shop floor factory personnel. For example, UK Patent Specification 1500850 describes a method of determining the biocide concentration of a liquid containing an aldehyde release biocide which comprises mixing a sample of the liquid, aqueous strong acid solution and a modified Schiff's reagent, and observing the colour developed. A kit for carrying out this method is also described. However, the Schiff's spot test for aldehydes has certain known deficiencies which may lead to inaccurate results, and, in addition, a relatively long period of time is required to carry out the test. Thus the Schiff's test is not particularly suitable for use on the shop floor by personnel unskilled in chemical analysis.

It is therefore an object of the present invention to provide a method for determining and/or monitoring the concentration of aldehyde release biocides in aqueous systems which is reasonably accurate and which can be carried out rapidly and easily by personnel with little chemical knowledge, for example, the factory personnel who normally operate the machines or apparatus in which the systems are used.

The method for determining the concentration of an aldehyde release biocide in an aqueous system according to the present invention comprises sampling a known quantity of liquid from the system, subjecting the sample to a colorimetric 3-methyl-benzothiazol-2-one hydrazone (MBTH) test procedure for the determination of aliphatic aldehydes and using the colour induced by the test procedure to determine the concentration of aliphatic aldehyde present in the sample characterised in that the aqueous system is an oil-water emulsion, the sample is diluted from 20 to 1000 times before subjecting it to the test procedure, the pH is adjusted before or during the test to the acid range and the determination of the aliphatic aldehyde content and thus the biocide content comprises matching the colour induced by the test procedure either when viewed through a yellow filter or in the presence in the sample of a yellow colouring agent with a colour scale showing the relationship between the colours obtained by applying the method to a series of samples containing the known concentrations of the biocide.

Further according to the present invention, a kit for determining the concentration of an aldehyde release biocide in an aqueous system, by employing the method as hereinbefore described, comprises (1) a container of an aqueous solution of a suitable oxidising agent; (2) a container of 3-methyl-benzothiazol-2-one hydrazone or a suitable acid addition salt thereof; (3) means for measuring out given quantities of the chemicals and a sample of the aqueous system; and (4) a standard colour scale calibrated for biocide concentration.

Preferably a number of containers in which the chemicals and sample may be mixed, are also included in the kit. The container of 3-methyl-benzothiazol-2-one hydrazone, or the acid addition salt thereof, may have the reagent in concentrated liquid or solid form (e.g. tablets) which requires diluting with or dissolving in distilled water. In such a case the kit may include a container in which such a solution may be prepared and stored.

2

O 016 578

The method of the present invention may be used to determine and/or monitor the concentration of any aldehyde preferably formaldehyde release type biocides in aqueous systems of the foregoing type. The method gives a semi quantitative estimation of the concentration of biocide present in the system. This estimation is sufficiently accurate for most control purposes. Examples of such systems and biocides have been given previously in this specification. The method is particularly suitable for use in factories to monitor the concentration of aldehyde release biocides in soluble oil/water emulsions, e.g. cutting oil emulsions, used in metal working machines. The concentration of biocide in the system can be maintained at any required value by using the present method to monitor the quantity of biocide present and then adding biocide to the system in accordance with the concentration determined.

The colorimetric MBTH test procedure for the determination of aldehydes in fluid systems is a known analytical procedure. There are a number of known techniques for carrying out the procedure and any of these techniques is suitable for use in the present invention. A description of the procedure with reference to one technique in which ferric chloride is used as the oxidising agent is given in Aldehydes—Photometric Analysis, E. Sawicki and C. R. Sawicki, Volume 1 at pages 59—69 and 231—233. The procedure is also disclosed in a paper entitled "The 3-Methyl-2-benzothiazolone Hydrazone Test" by E. Sawicki et al, published in Analytical Chemistry, Volume 33, No. 1 January 1961 pages 93 to 93, USA. Another suitable oxidising agent is hydrogen peroxide. The test is based on the reaction of aliphatic aldehydes with 3-methyl-benzothiazol-2-one hydrazone to form the monoazines which are then oxidised by a suitable oxidising agent to the diazo compounds which are blue chromogens. The intensity of the blue colour thus induced is taken as a measure, of the quantity of aldehyde present in the original sample.

We have found that the sensitivity of the test can be improved by broadening the colour spectrum induced to embrace the following range of colours; yellows, greens and blues. The colour spectrum can be broadened either by viewing the blue colour induced by the MBTH test procedure through a yellow filter or by the presence in the solution tested of a yellow colouring agent or the use of a yellow filter in combination with a yellow colouring agent.

When a yellow colouring agent is used it can be added to the sample prior to application of the MBTH test procedure or at any time during or after application of the procedure.

Any yellow colouring agent which does not interfere with the test procedure can be used. The colouring agent can be and preferably is the ferric chloride used as the oxidising agent. Examples of other colouring agents are: acriflavin, tartrazine, food yellow 3, and quinoline yellow.

The colour scale used for determining the concentration of biocide present in the test sample can be compiled by applying the method to a series of samples containing known concentrations of the biocide. The concentrations of the biocide should be of a similar order to the concentration required in the aqueous system. The colour scale can be, for example, in the form of a chart, or a series of colour slides or transparencies. The colour of the test samples can be readily matched with a colour on the colour scale given by a known concentration of the biocide and hence the concentration of biocide in the sample is determined.

The test sample must be diluted in order that the presence of the oil in the aqueous system does not interface with the colour induced by the MBTH test procedure. The emulsion should be diluted by an order of at least ×20 and conveniently about ×100. The upper limit is not critical provided a sufficient concentration of biocide is present to develop a colour. A practical upper limit may be ×1000.

In order to obtain an accurate determination of the quantity of biocide in the test sample the release of aldehyde should be at a maximum. Release of aldehyde from the biocide can be facilitated by adjusting the pH of the sample to the acid range either before or during the test. Most suitably the pH can have a value in the range 3 to 5. The use of MBTH in the acid addition salt form, e.g. the hydrochloride (MBTH · HCl), is usually sufficient to induce a pH which ensures maximum release of the aldehyde.

The present method is particularly suitable for use in the factory by personnel unskilled in chemical analysis for the routine monitoring of the biocide present in oil/water emulsions used in metal working machines. For most applications it is usually desirable to maintain the aldehyde concentration in aqueous systems of this type at a value in the range 100 to 1000 parts per million.

The method of the present invention is illustrated by the following examples.

Example 1

A soluble oil cutting fluid concentrate having the following composition was prepared. Spindle oil—78.5 percent; additive comprising emulsifiers; coupling agents and corrosion inhibitors—20 per cent and Cinon—1.5 percent. The percentages are by weight. Cinon is the Registered Trade Mark of a formaldehyde release biocide used to suppress micro-organisms in aqueous systems sold by Bacillolfabrik Dr. Bode & Co.

Known amounts of the cutting fluid concentrate were dispersed in water to give a series of aqueous systems containing 100, 250, 500 and 750 parts per million of Cinon. The pH values of these systems ranged from 7 to 9. 50 $\mu$l of each system was then sampled by means of a syringe and the samples dispersed with shaking in 5 millilitres of distilled water. Each dispersion was then transferred to a vial containing 5 milligrams of MBTH HCl and each vial shaken until all the solid had dissolved. Two

3

50 $\mu$l drops of a 2 percent by weight anhydrous ferric chloride aqueous, yellow solution were then added to each vial and the vials again shaken and then allowed to stand for 3 minutes. The colour thus induced in each system was then matched with a colour on a chart showing the relationship between standard Dulux Trade Colours obtained by applying the foregoing procedure to a further series of standard samples containing 100, 200, 400 and 800 parts per million of Cinon. The colours thus matched corresponded closely with the concentrations of Cinon in the aqueous systems. The colours in the chart are given tin Table 1.

TABLE 1

| | Quantity of Cinon in standard sample (parts per million) | | | | |
|---|---|---|---|---|---|
| | Blank | 100 | 200 | 400 | 800 |
| Standard Samples (Dulux Trade Colours) BS.4800 | Yellow | Lido | Larkspur | Cornflower | Trafalgar |
| BS No. | | 16E53 | 18E51 | 20E51 | 20D45 |

Example 2

The present method was used in a factory on the shop floor to monitor the quantity of biocide present in an aqueous cutting oil emulsion used in the sumps of two machine tools A and B. The cutting oil emulsion consisted of 1 part by volume of a soluble cutting oil concentrate containing 1.5 percent by weight of the biocide Cinon diluted with 20 parts by volume of water. Cinon is the Registered Trade Mark of a formaldehyde release biocide sold by Bacillolfabrik Dr. Bode & Co. The cutting oil emulsion in machine tool A was monitored over a period of 20 weeks and the emulsion in machine tool B was monitored over a period of 16 weeks. The intervals between sampling are given in Table 2. The quantity of biocide in each sample was determined in accordance with the method described in Example 1. The data thus obtained is given in Table 2. In relation to machine tool A the biocide content of the emulsion was maintained by topping up with further emulsion containing the biocide. In relation to machine tool B the method was used to determine the quantity of biocide present in the system without attempting to maintain the biocide concentration. In addition to the biocide determination each sample was subjected to a microbiological analysis to determine the number of aerobic bacteria present. The bacterial counts thus obtained are given in Table 2 below.

4

TABLE 2

| Week Number | Cutting oil emulsion: Maching tool A | | Cutting oil emulsion: Machine tool B | |
|---|---|---|---|---|
| | Residual biocide * content ppm | Aerobic bacteria (number/ml) | Residual biocide * content ppm | Aerobic bacteria (number/ml) |
| 1 | 800 | <100 | 800 | <100 |
| 2 | 600 | <100 | 800 | <100 |
| 4 | 800 | <100 | <200 | $6.7 \times 10^5$ |
| 6 | 700 | <100 | <100 | $1.0 \times 10^7$ |
| 8 | >600 | <100 | <100 | $3.0 \times 10^7$ |
| 10 | >600 | <100 | <100 | $3.7 \times 10^6$ |
| 12 | 300 | <100 | <100 | $1.0 \times 10^8$ |
| 14 | 600 | <100 | no sample | no sample |
| 16 | 600 | <100 | <100 | $1.6 \times 10^7$ |
| 18 | 600 | <100 | | ** |
| 20 | >400 | <100 | | |

\* Estimated by the method described in Example 1
\*\* Fluid failed due to generation of foul odours

It can be seen from the data given in Table 2 that, by monitoring the concentration of biocide and adding biocide to the system in accordance with the concentration determined, the bacterial population was effectively controlled in the cutting oil emulsion in machine tool A thus prolonging the life of the emulsion without exposing the operatives to a potential health hazard due to the build up of high formaldehyde concentrations. On the other hand, the biocide content of the cutting oil emulsion in machine tool B decreased with time with an accompanying increase in the bacterial population until the emulsion failed due to bacterial spoilage.

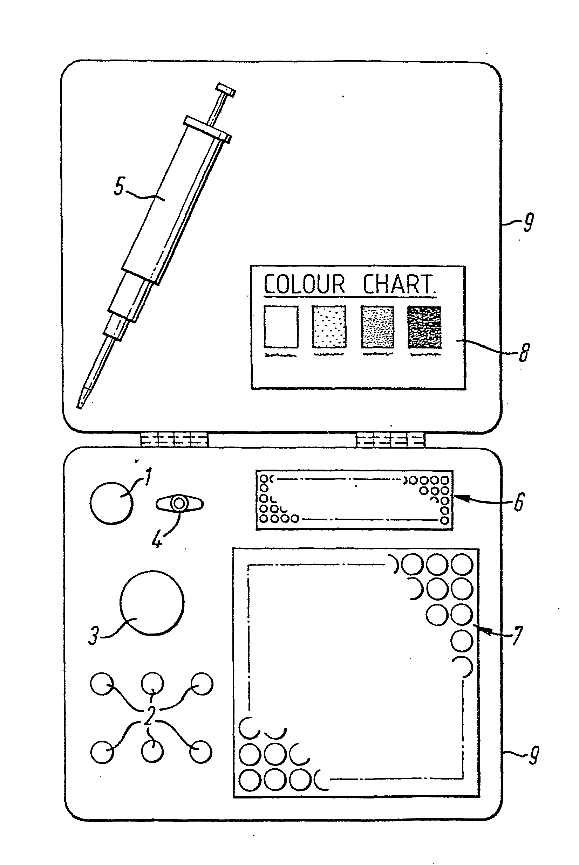

A kit, and a procedure for employing the kit, for the determination of aldehyde release biocides when present in aqueous systems, according to the present invention, is illustrated with reference to the accompanying drawing, which shows a top plan view of the opened test kit case.

The illustrated kit comprises:

(1) a 50 ml screw capped, amber glass bottle containing a 6% by weight solution of hydrated ferric chloride in distilled water, 1.
(2) six vials each containing 0.1 g of MBTH HCl 2.
(3) a 100 ml screw capped bottle 3, in which the MBTH HCl contained in one of the vials 2 may be made up to a 0.1% by weight solution with distilled water, and stored.
(4) a 5 ml measuring syringe 4.
(5) a 50 $\mu$l fixed volume pipetter 5.
(6) 120 disposable Pippetter tips 6.
(7) 100 7 ml screw capped plastic vials 7.
(8) a standard colour chart 8 compiled by applying the method to a series of samples of known concentrations of the biocide.
(9) a container 9 into which all the elements of the kit 1—8 are fitted.

A typical procedure for the use of the kit is as follows.

A 0.1% by weight solution of MBTH HCl is prepared by dissolving the contents of one of the vials 2 in 100 ml of distilled water in the 100 ml bottle 3, without heating. The MBTH HCl in the vials 2 may be a powder or may be in the form of a tablet. Conventional drug packaging may be used in place of the vials 2 if the MBTH HCl is used in tablet form. The 100 ml solution, which is sufficient for 20 tests, may be stored at room temperature or preferably in a refrigerator. If the solution develops a brown colouration or is not used within one month, a fresh solution should be prepared.

5 ml of the 0.1% by weight MBTH HCl solution is transferred from the bottle 3 into a 7 ml vial 7, using the 5 ml measuring syringe 4. To this is added 50 $\mu$l of a representative sample of the aqueous system to be tested, using the fixed volume Pipetter 5, fitted with a new Pipetter tip 6. The vial cap is replaced and the vial shaken to disperse the test sample in the MBTH HCl solution. The Pippetter tips 6 and the 7 ml vials 7 are disposable and a new tip and vial should be used for each test sample.

Using the 50 $\mu$l fixed volume Pippeter 5, fitted with a new tip 6, 50 $\mu$l of the 6% by weight ferric chloride solution, from the bottle 1, are added to the contents of the vial 7. Replacing the screw cap, the vial 7 is shaken to disperse the ferric chloride solution. Preferably the ferric chloride solution is added to the contents of the vial 7, between fifteen minutes and one hour after mixing the test sample and the MBTH HCl solution. The ferric chloride solution is both the oxidising agent and the yellow colouring agent.

The vial is allowed to stand for three minutes while the colour develops. This colour fades with time so it should be matched with a colour on the colour chart 8, between three and ten minutes after adding the ferric chloride solution, in order to determine the concentration of the biocide in the sample.

## Claims

1. A method for determining the concentration of an aldehyde release biocide in an aqueous system comprising sampling a known quantity of liquid from the system, subjecting the sample to a colorimetric 3-methyl-benzothiazol-2-one hydrazone (MBTH) test procedure for the determination of aliphatic aldehydes and using the colour induced by the test procedure to determine the concentration of aliphatic aldehyde present in the sample characterised in that the aqueous system is an oil-water emulsion, the sample is diluted from 20 to 1000 times before subjecting it to the test procedure, the pH is adjusted before or during the test to the acid range and the determination of the aliphatic aldehyde content and thus the biocide content comprises matching the colour induced by the test procedure, either when viewed through a yellow filter or in the presence in the sample of a yellow colouring agent, with a colour scale showing the relationship between the colours obtained by applying the method to a series of samples containing known concentrations of the biocide.

2. A kit for determining the concentration of an aldehyde release biocide in an aqueous system, by employing the method as claimed in claim 1, characterised in that the kit comprises (1) a container of an aqueous solution of a suitable oxidising agent; (2) a container of 3-methyl-benzothiazol-2-one hydrazone, or a suitable acid addition salt thereof; (3) means for measuring out given quantities of the chemicals and a sample of the aqueous system; (4) a standard colour scale calibrated for biocide concentration.

3. A kit according to claim 2 which also includes a number of containers in which the chemicals and sample may be mixed.

4. A kit according to either of claims 2 or 3 in which the acid addition salt is 3-methyl-benzothiazol-2-one hydrazone hydrochloride.

## Revendications

1. Procédé de détermination de la concentration d'un biocide libérateur d'aldéhyde dans un système aqueux, consistant à prélever comme échantillon une quantité connue de liquide du système, à soumettre l'échantillon à une processus d'essai colorimétrique à la 3-méthyl-benzo-thiazole-2-one-hydrazone (MBTH) pour la détermination des aldéhydes aliphatiques et à utiliser la couleur engendrée par le processus d'essai pour déterminer la concentration d'aldéhyde aliphatique présent dans l'échantillon, caractérisé par le fait que le système aqueux est une émulsion eau-huile, en ce que l'échantillon est dilué de 20 à 1000 fois avant de le soumettre au processus d'essai, en ce que l'on ajuste le pH dans la gamme acide avant ou pendant l'essai et en ce que la détermination de la teneur en aldéhyde aliphatique et donc de la teneur en biocide consiste à faire correspondre la couleur engendrée par le processus d'essai, soit lorsqu'on regarde à travers un filtre jaune soit en présence d'un colorant jaune dans l'échantillon, avec une échelle de couleurs montrant la relation obtenue entre les couleurs en appliquant la méthode à une série d'échantillons contenant des concentrations connues du biocide.

2. Trousse pour la détermination de la concentration d'un biocide libérateur d'aldéhyde dans un système aqueux, par utilisation du procédé selon la revendicaiton 1, caractérisée par le fait qu'elle comprend (1) un récipient d'une solution aqueuse d'un oxydant approprié, (2) un récipient de 3-méthyl-benzothiazole-2-one-hydrazone ou d'un sel d'addition d'acide approprié de celle-ci, (3) des moyens permettant de mesurer des quantités données des agents chimiques et d'un échantillon du système aqueux, et (4) une échelle de couleurs normalisées étalonnée pour la concentration en biocide.

3. Trousse selon la revendication 2, qui comprend aussi un certain nombre de récipients dans lesquels on peut mélanger les agents chimiques et l'échantillon.

4. Trousse selon l'une des revendications 2 et 3, dans laquelle le sel d'addition d'acide est le chlorhydrate de 3-méthyl-benzothiazole-2-one-hydrazone.

# 0016578

## Patentansprüche

1. Verfahren zur Bestimmung der Konzentration eines Aldehyd abgebenden Biozids in einem wäßrigen System, wobei also Probe dem System eine bekannte Flüssigkeitsmenge entnommen wird, die Probe zur Bestimmung aliphatischer Aldehyde einem bekannten kolorimetrischen Testverfahren mittels 3-Methyl-benzothiazol-2-on-hydrazon (MBTH) unterworfen wird und die bei diesem Testverfahren erzeugte Färbung zur Bestimmung der Konzentration des in der Probe vorhandenen aliphatischen Aldehyds verwendet wird, dadurch gekennzeichnet, daß das wäßrige System eine Öl-Wasser-Emulsion ist, die Probe, bevor sie dem Testverfahren unterworfen wird, auf das Zwanzig- bis Eintausendfache verdünnt wird, der pH vor dem Test oder während desselben auf den sauren Bereich eingestellt wird und die Bestimmung des Gehalts an aliphatischem Aldehyd und damit des Biozid-Gehalts den Vergleich der mittels des Testverfahrens gebildeten Färbung, entweder beim Betrachten durch ein Gelbfilter oder in Anwesenheit eines gelben Färbemittels in der Probe, mit einer Fabrskala umfaßt, die die Beziehung zwischen den bei Anwendung des Verfahrens auf eine Reihe von Proben mit bekannten Konzentrationen des Biozids erhaltenen Farben wiedergibt.

2. Testsatz sur Bestimmung der Konzentration eines Aldehyd abgebenden Biozids in einem wäßrigen System durch Anwendung des Verfahrens nach Anspruch 1, dadurch gekennzeichnet, daß er

(1) einen Behälter mit einer wäßrigen Lösung eines geeigneten Oxidationsmittels,
(2) einen Behälter mit 3-Methyl-benzothiazol-2-onhydrazon oder einem geeigneten Säureadditionssalz desselben,
(3) Mittel zum Abmessen vorgegebener Mengen der Chemikalien und einer Probe des wäßrigen Systems und
(4) eine für die Biozid-Konzentration kalibrierte Standard-Farbskala enthält.

3. Testsatz nach Anspruch 2, dadurch gekennzeichnet, daß er weiterhin eine Anzahl Gefäße enthält, in denen die Chemikalien und die Probe miteinander vermischt werden können.

4. Testsatz nach einem der Ansprüche 2 oder 3, dadurch gekennzeichnet, daß das Säureadditionssalz 3-Methyl-benzothiazol-2-on-hydrazon-hydrochlorid ist.